# FASCICULE DE BREVET EUROPEEN

(11) **EP 2 135 576 B1**
(45) Date de publication et mention de la délivrance du brevet: **02.09.2015**
(21) Numéro de dépôt: 09163299.2
(22) Date de dépôt: 19.06.2009
(51) Int. Cl.: A61F 2/30, G06T 17/05, A61B 19/00

(54) **Procédé de modélisation d'une surface glénoïdienne d'une omoplate, dispositif d'implantation d'un composant glénoïdien d'une prothèse d'épaule, et procédé de fabrication d'un tel composant**
Modellierungsverfahren einer Schultergelenksoberfläche eines Schulterblatts, Implantierungsvorrichtung einer Schultergelenkskomponente einer Schulterprothese und Herstellungsverfahren einer solchen Komponente
Method for modelling a glenoidal surface of a scapula, device for implanting a glenoidal component of a shoulder prosthesis, and method for manufacturing such a component

(30) Priorité: 20.06.2008 FR 0854092
(43) Date de publication de la demande: 23.12.2009
(73) Titulaire: TORNIER, 38330 Montbonnot-Saint-Martin (FR)
(72) Inventeur: Tornier, Alain, 38330, SAINT ISMIER (FR); Henry, Delphine, 38330, SAINT-ISMIER (FR)
(74) Mandataire: Grand, Guillaume

(56) Documents cités:
- EP-A- 1 249 213
- EP-A- 1 563 810
- EP-A- 1 862 151
- WO-A-02/061688
- FR-A- 2 579 454
- FR-A- 2 859 099
- US-A1- 2005 065 628

## Description

La présente invention concerne un procédé de modélisation d'une surface glénoïdienne d'une omoplate. Elle concerne également un dispositif chirurgical pour implanter un composant glénoïdien d'une prothèse d'épaule. Elle concerne en outre un procédé de fabrication d'un tel composant.

Le remplacement de la surface articulaire glénoïdienne de l'omoplate d'un être humain par un composant prothétique glénoïdien concave d'une prothèse d'épaule est une opération chirurgicale délicate, notamment en raison de l'environnement ligamentaire de l'épaule. On constate que, selon la géométrie articulaire et/ou la position d'implantation d'un tel composant glénoïdien, des risques de décèlement du composant existent en raison de la modification des efforts appliqués à ce composant lors des mouvements ultérieurs de l'épaule prothésée.

Actuellement, les chirurgiens orthopédiques choisissent un tel composant glénoïdien parmi une gamme d'implants, présentant des géométries, notamment des tailles, légèrement différentes les unes des autres, puis estiment empiriquement la position d'implantation du composant glénoïdien choisi, en appréciant visuellement la géométrie de la surface glénoïdienne du patient opéré : le chirurgien essaie de choisir et d'implanter le composant prothétique sur l'omoplate de manière à ce que ce composant reproduise la surface articulaire glénoïdienne d'origine du patient. Toutefois, cette méthode est imprécise et, surtout, très peu satisfaisante lorsque la glène d'origine du patient est trop dégénérative pour fournir une indication directement exploitable par l'observation chirurgicale.

Dans le domaine de la chirurgie orthopédique de la hanche, EP-A-1 563 810 propose un système d'implantation d'une prothèse totale de la hanche, assistée par ordinateur.

Le but de la présente invention est de proposer un procédé de modélisation d'une surface glénoïdienne d'une omoplate, en vue d'aider le chirurgien à optimiser la position d'implantation d'un composant glénoïdien et/ou en vue de fabriquer un composant glénoïdien davantage adapté à une omoplate à prothéser, notamment en présence d'une glène opérée dans un état de dégénérescence avancée.

A cet effet, l'invention a pour objet un procédé de modélisation d'une surface glénoïdienne d'une omoplate, tel que défini à la revendication 1.

L'idée à la base de l'invention est liée au constat selon lequel, dans la très grande majorité des cas chirurgicaux, la dégénérescence de la glène d'un patient est située exclusivement dans la partie haute et postérieure de la surface glénoïdienne. Dans ces conditions, l'invention se propose d'utiliser exclusivement une partie située le plus bas de la surface glénoïdienne dégénérative, pour obtenir par modélisation une surface glénoïdienne théorique aussi proche que possible de la surface glénoïdienne avant dégénérescence, à l'aide d'au moins une portion d'ellipsoïde imaginaire judicieusement calculée, comme défini ci-dessus. En pratique, la méthode de modélisation conforme à l'invention est mise en oeuvre par des moyens ad hoc, en particulier informatiques.

Le fait d'estimer par modélisation cette surface glénoïdienne théorique présente des intérêts remarquables, à la fois et de manière indépendante, pour optimiser la position d'implantation d'un composant glénoïdien pré-existant et pour fabriquer un composant glénoïdien spécifiquement adapté à l'omoplate du patient, comme exposé ci-après plus en détail. Ainsi, l'invention a également pour objets un dispositif chirurgical et une méthode chirurgicale d'implantation d'un composant glénoïdien d'une prothèse d'épaule, ainsi qu'un procédé de fabrication d'un composant glénoïdien pour une omoplate, tels que définis plus bas.

D'autres caractéristiques du procédé de modélisation conforme à l'invention, précisant des aspects avantageux de ce procédé, prises isolément ou selon toutes les combinaisons techniquement possibles, sont spécifiées aux revendications dépendantes 2 à 6.

Comme annoncé plus haut, l'invention a en outre pour objet un dispositif chirurgical d'implantation d'un composant glénoïdien d'une prothèse d'épaule, tel que défini à la revendication 7.

Ainsi, grâce, d'une part, à la surface glénoïdienne théorique fournie par le procédé de modélisation défini plus haut et, d'autre part, à une information relative à un point de référence bas, que le chirurgien peut obtenir directement de l'extrémité inférieure en principe non altérée de la surface glénoïdienne dégénérative du patient, le chirurgien peut baser ses gestes d'implantation d'un composant glénoïdien sur des données satisfaisantes en ce qui concerne la géométrie de la surface glénoïdienne avant dégénérescence du patient opéré. Le chirurgien est ainsi en mesure d'améliorer la configuration d'implantation de ce composant glénoïdien, et ce au cours de l'intervention chirurgicale.

Des caractéristiques avantageuses, précisant la mise en oeuvre du dispositif conforme à l'invention, prises isolément ou selon toutes les combinaisons techniquement possibles, sont spécifiées aux revendications dépendantes 8 à 11.

On définit également ici une méthode chirurgicale d'implantation d'un composant glénoïdien d'une prothèse d'épaule, dans laquelle :
- on repère dans l'espace l'omoplate d'un patient opéré,
- on cartographie la surface glénoïdienne de cette omoplate, en utilisant des données acquises par palpation de l'omoplate et/ou des données extraites d'images pré-opératoires et/ou de données fournies par une base de données préétablies,
- on modélise la surface glénoïdienne conformément au procédé de modélisation défini plus haut, de sorte que, à partir des données de cartographie, on obtient une surface glénoïdienne théorique de l'omoplate du patient opéré,
- on détermine la position dans l'espace d'un point de référence bas pour implanter le composant glénoïdien, à partir des données liées à la cartographie de l'extrémité inférieure de la surface glénoïdienne, et
- on implante le composant glénoïdien dans une configuration spatiale déterminée au moins à partir de la surface glénoïdienne théorique et du point de référence bas.

Cette méthode chirurgicale peut être mise en oeuvre par le dispositif d'implantation tel que défini plus haut.

Par ailleurs, cette méthode présente avantageusement des aspects additionnnels qui correspondent respectivement à la mise en oeuvre pratique des caractéristiques additionnelles avantageuses listées ci-dessus pour le dispositif d'implantation.

Comme annoncé plus haut, l'invention a en outre pour objet un procédé de fabrication d'un composant glénoïdien d'une prothèse d'épaule pour une omoplate, tel que défini à la revendication 12.

Le procédé de fabrication conforme à l'invention permet, en quelque sorte, d'obtenir un implant glénoïdien « sur mesure », dans le sens où sa face articulaire reproduit aussi fidèlement que possible la surface glénoïdienne, avant sa dégénérescence, de l'omoplate à prothéser. Le confort articulaire pour le patient est alors remarquable.

Bien entendu, pour une même omoplate traitée, il est avantageusement possible de fabriquer un composant glénoïdien « sur mesure », grâce au procédé de fabrication conforme à l'invention, puis d'implanter ce composant glénoïdien à l'aide du dispositif d'implantation défini plus haut, c'est-à-dire conformément à la méthode d'implantation également définie plus haut.

Un aspect complémentaire avantageux du procédé de fabrication conforme à l'invention est spécifié à la revendication dépendante 13.

L'invention sera mieux comprise à la lecture de la description qui va suivre, donné uniquement à titre d'exemple et faite en se référant aux dessins sur lesquels :
- la figure 1 est une vue schématique d'un dispositif d'implantation selon l'invention, appliqué à l'omoplate d'un patient à opérer en vue d'y implanter un composant glénoïdien ;
- les figures 2 à 4 sont des sections schématiques, dans un plan frontal au patient, de l'omoplate en cours d'opération à l'aide du dispositif de la figure 1 ; et
- la figue 5 est une vue schématique en élévation d'un composant glénoïdien fabriqué conformément au procédé de fabrication selon l'invention.

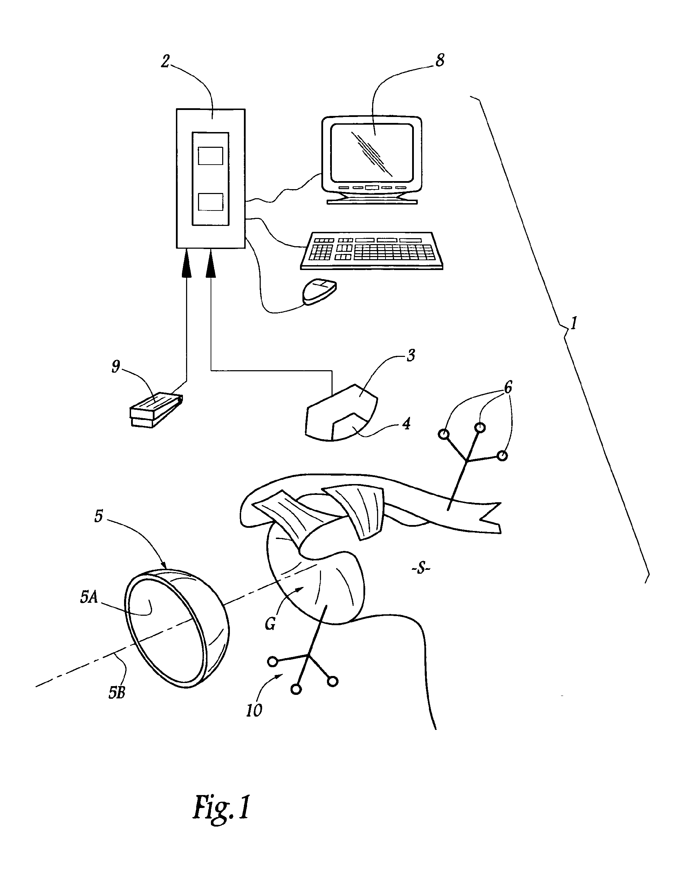

Le dispositif chirurgical 1 de la figure 1 comprend un ordinateur 2 associé à une unité d'émission et de réception d'un rayonnement infrarouge. Cette unité comporte un capteur 3 relié à l'ordinateur et une source d'alimentation infrarouge 4 couvrant le champ opératoire dans lequel est représenté en partie l'omoplate S d'un patient à traiter.

L'omoplate S est associée, entre autres, à des tendons et ligaments et délimite, sur son côté latéral, une surface glénoïdienne G. Cette surface glénoïdienne G est dégénérative, c'est-à-dire qu'elle est partiellement endommagée par usure en raison de l'âge avancé et/ou d'une pathologie du patient opéré. Comme expliqué ci-après en détail, le dispositif 1 est adapté pour aider un chirurgien à implanter, en remplacement de la surface glénoïdienne dégénérative G, un composant prothétique glénoïdien 5, connu en soi. Dans l'exemple considéré ici, ce composant glénoïdien 5 présente une forme globale de cupule et délimite une face articulaire 5A de géométrie sensiblement sphérique, définissant un axe de révolution 5B.

Le composant prothétique 5 décrit juste ci-dessus n'est donné qu'à titre d'exemple et d'autres composants glénoïdiens prothétiques, de géométries et/ou de natures différentes, peuvent être implantés au moyen du dispositif 1, suivant la méthode chirurgicale d'implantation décrite plus loin.

Pour permettre à l'ordinateur 2 de repérer dans l'espace l'os de l'omoplate S, le dispositif 1 comporte un groupe de marqueurs 6 qui renvoient, de façon passive, le rayonnement infrarouge en direction du capteur 3. Ce groupe de marqueurs 6 forme un système de marquage tridimensionnelle permettant à l'ensemble ordinateur 2 - capteur 3 de suivre dans l'espace la position et le déplacement de l'omoplate. L'utilisation de tels marqueurs est bien connue dans le domaine de l'orthopédie assistée par ordinateur, par exemple par le document EP-A-1 249 213, de sorte que ces marqueurs ne seront pas décrits ici plus avant.

L'ordinateur 2 est également associé à un écran vidéo 8 à même d'afficher des informations utiles au chirurgien, notamment les informations relatives au repérage de l'omoplate S et d'autres données décrites plus loin, de préférence sous forme de représentations graphiques comme détaillé ci-après. Le dispositif 1 comporte également des moyens de commande 9, par exemple sous forme d'une pédale actionnable par le pied du chirurgien.

Le dispositif chirurgical 1 comporte en outre d'autres composants qui vont être détaillés ci-après lors de la description d'un exemple détaillé d'utilisation de ce dispositif en vue de l'implantation du composant glénoïdien 5. Par convention dans tout le présent document, les termes de positionnement dans l'espace, tels que les termes « haut », « bas », « vertical », « horizontal », etc., s'entendent dans leur sens anatomique, c'est-à-dire par rapport au patient opéré considéré comme se tenant debout sur une surface plane.

Dans un premier temps, le chirurgien incise les parties molles de l'épaule du patient et recueille un certain nombre de données relatives, entre autres, à la géométrie anatomique de l'os de l'omoplate S du patient. A cet effet, différents moyens d'acquisition de ces données sont envisageables. A titre d'exemple, le chirurgien utilise un palpeur 10 repéré par l'ensemble ordinateur 2 - capteur 3 et préalablement étalonné. Ce palpeur est amené sur des lieux remarquables de l'omoplate, notamment sur toute la surface glénoïdienne G, et, par actionnement de la pédale de commande 9, le chirurgien fait enregistrer à l'ordinateur 2 la position du palpeur 10. A partir de ces données et, le cas échéant, de données préenregistrées relatives à une géométrie de base de l'omoplate d'un être humain, l'ordinateur 2 est capable de dresser une cartographie tridimensionnelle de la surface glénoïdienne dégénérative G de cette omoplate.

A titre de variante des différentes possibilités pour acquérir les données de cartographie relatives à la géométrie anatomique de la surface glénoïdienne G, on peut envisager d'extraire de telles données depuis des images pré-opératoires de l'omoplate S du patient, par exemple des images de scanner. De telles données peuvent d'ailleurs être combinées à des données obtenues par palpation comme ci-dessus, en les associant éventuellement à des données prédéterminées fournies par des bases de données disponibles dans le domaine de la chirurgie de l'épaule.

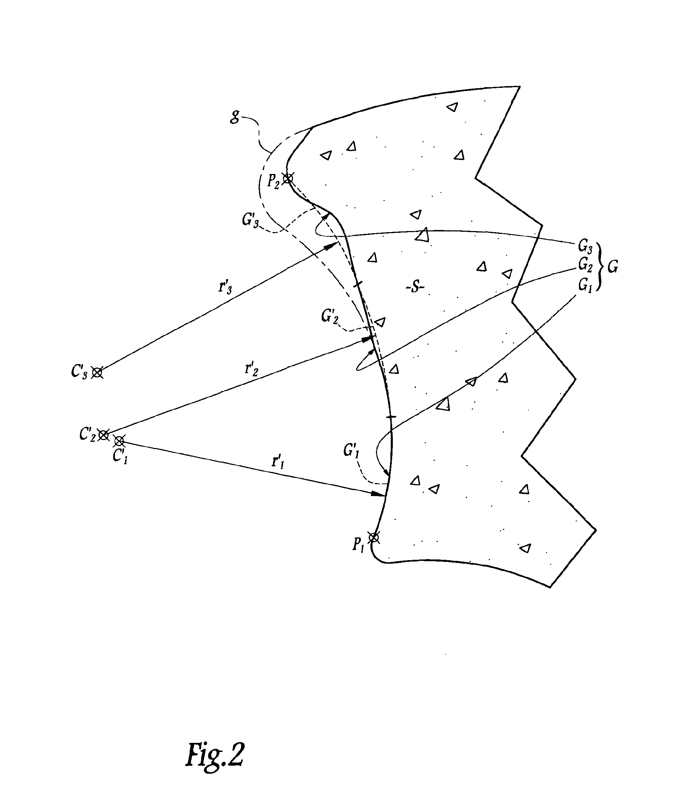

A la fin de ce premier temps, l'ordinateur 2 affiche sur l'écran 8 la cartographie réalisée, notamment à des fins de contrôle visuel par le chirurgien. Cet affichage est en particulier réalisé dans un plan frontal au patient, passant par les points de cartographie appartenant à la surface glénoïdienne G et respectivement situés le plus bas et le plus haut, comme illustré par la figure 2 sur laquelle les deux points de cartographie précités sont respectivement référencés P₁ et P₂.

La figure 2 montre bien, ici de manière exagérée à des fins de compréhension du présent document, que la dégénérescence de la surface glénoïdienne G est essentiellement située dans la partie haute de cette surface, tout particulièrement dans le tiers supérieur de cette surface. A titre de comparaison pour le lecteur, la surface glénoïdienne avant dégénérescence est indiquée en traits mixtes et pointée par la référence g : par comparaison des traces respectives des surfaces g et G dans le plan de la figure 2, on constate que la surface G a subi une dégénérescence par usure bien plus prononcée dans sa partie haute que dans sa partie basse, qui est restée quasiment intacte.

Dans un deuxième temps, le chirurgien commande l'ordinateur 2 pour qu'il traite les données de cartographie obtenues lors du premier temps opératoire. Des moyens informatiques intégrés à l'ordinateur 2 traitent, soit de manière complètement préétablie, soit en intégrant des paramètres choisis de manière discrétionnaire par le chirurgien, les données de cartographie de manière à répartir les points de cartographie de la surface glénoïdienne G en trois groupes distincts, correspondant à des parties respectives G₁, G₂ et G₃ de cette surface G, qui se succèdent de bas en haut suivant une direction verticale.

Par exemple, les trois parties de surface G₁, G₂ et G₃ présentent une dimension verticale identique.

Les moyens informatiques précités traitent ensuite, de manière indépendante, chacun des trois groupes de données de cartographie respectivement associés aux parties de surface G₁, G₂ et G₃, pour modéliser une portion de sphère imaginaire G'₁, G'₂ et G'₃, indiquée en pointillés à la figure 2, qui géométriquement coïncide globalement avec la partie de surface correspondante G₁, G₂ et G₃. En pratique, la position du centre C'₁, C'₂, C'₃ et la valeur du rayon r'₁, r'₂, r'₃ de chaque portion de sphère G'₁, G'₂, G'₃ sont calculées par les moyens informatiques précités de manière que cette portion de sphère passe par le plus grand nombre de points cartographiés pour la partie de surface correspondante G₁, G₂ et G₃, étant entendu que chacune de ces portions de sphère est considérée comme passant par un de ces points cartographiés lorsque l'écart multidirectionnel entre cette portion de sphère et ce point est nul ou, tout au moins, inférieur à une valeur prédéterminée. D'autres méthodes mathématiques pour déterminer les caractéristiques géométriques des portions de sphère G'₁, G'₂ et G'₃ peuvent être employées en variantes.

Avantageusement, l'ordinateur 2 affiche sur l'écran 8, à l'attention du chirurgien, tout ou partie des portions de sphère modélisées G'₁, G'₂, G'₃, en particulier leur centre C'₁, C'₂, C'₃ , simultanément à l'affichage de la cartographie de la glène dégénérative G comme montré sur la figure 2.

Sur cette figure 2, on constate ainsi que, dans la mesure où les parties de surface inférieure G₁ et intermédiaire G₂ ne sont pas ou ne sont que peu dégénératives, les centres C'₁ et C'₂ de leur portion de sphère associée G'₁ et G'₂ obtenue par modélisation sont très proche l'un de l'autre comparativement au centre C'₃ de la portion de sphère G'₃ modélisée à partir de la partie de surface supérieure G₃.

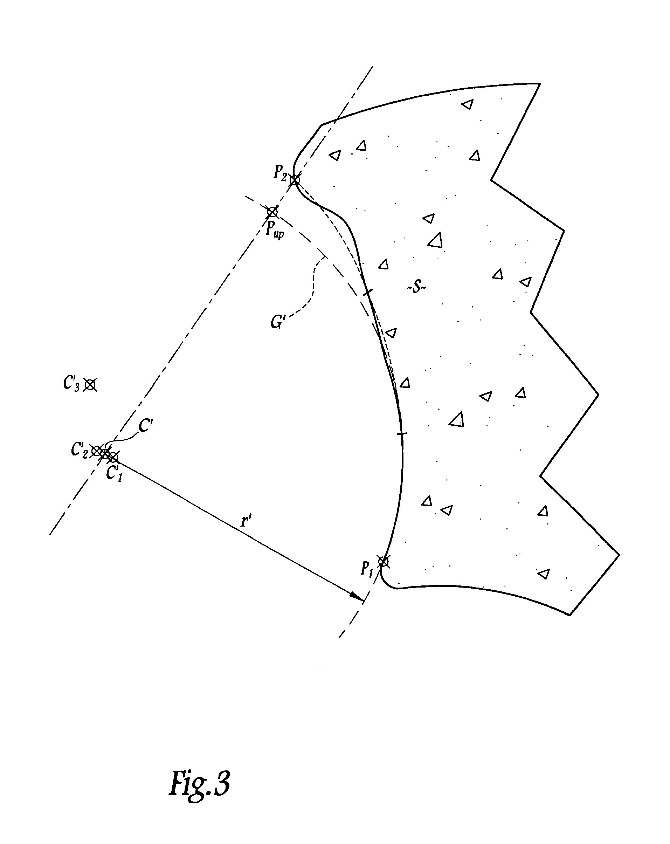

Dans un troisième temps, de préférence sous réserve que le chirurgien valide les résultats de modélisation obtenus jusqu'ici et affichés sur l'écran 8, en particulier que les centres modélisés C'₁,et C'₂ sont effectivement proches l'un de l'autre par comparaison au centre C'₃, les moyens informatiques de l'ordinateur 2 construisent, par calcul, une surface glénoïdienne théorique sphérique G', qui est centré sur un centre C' et qui présente un rayon r', dont, respectivement, la position et la valeur sont calculées à partir des positons des centres C'₁ et C'₂ et à partir des valeurs des rayons r'₁ et r'₂. A titre d'exemple, comme illustré à la figure 3, le centre C' est calculé en tant que barycentre des centres C'₁ et C'₂ tandis que le rayon r' est calculé comme étant la moyenne des rayons r'₁ et r'₂. Plus généralement, un point essentiel de l'invention consiste, à cette étape, à utiliser les données géométriques modélisées pour la région de la surface glénoïdienne G la moins dégénérative, autrement dit la moins usée, c'est-à-dire les parties de surface inférieure G₁ et intermédiaire G₂. De la sorte, on comprend que la surface glénoïdienne théorique G' ainsi calculée correspond à une estimation fiable de toute la surface glénoïdienne avant dégénérescence g. On comprend également que, en pratique, on peut se passer de calculer les caractéristiques géométriques de la portion de sphère G'₃ si on renonce à afficher cette dernière sur l'écran 8 et à la comparer aux portions de sphère G'₁ et G'₂.

L'ordinateur 2 détermine alors ensuite la position du point d'intersection entre cette surface glénoïdienne théorique G' et la droite radiale à cette surface passant par le point de cartographie P₂, c'est-à-dire la droite passant par les points C' et P₂. Comme représenté sur la figure 3, ce point d'intersection est référencé Pᵤₚ. On comprend que ce point Pᵤₚ correspond à une estimation fiable du point d'extrémité supérieure de la surface glénoïdienne avant dégénérescence g. Le point Pᵤₚ est de préférence affiché par l'ordinateur 2 sur l'écran 8 à l'attention du chirurgien, pour contrôle visuel.

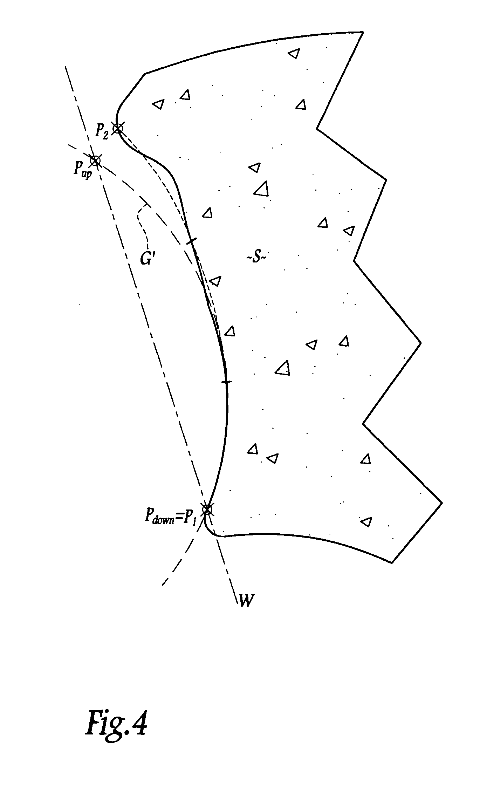

Dans un quatrième temps, l'ordinateur 2 fournit au chirurgien, en l'affichant sur l'écran 8, un plan W d'implantation du composant glénoïdien 5, dont la trace est visible à la figure 4 et qui passe à la fois par le point précité Pᵤₚ et un point P_{down} déterminé par l'ordinateur 2. Avantageusement, ce point P_{down} correspond au point cartographié P₁.

L'important est que le plan d'implantation W correspond à un plan préférentiel pour implanter le composant glénoïdien 5 dans le sens où il assure au chirurgien un comportement biomécanique du composant glénoïdien 5 proche, voire analogue, au comportement de la surface glénoïdienne avant dégénérescence g si le composant 5 est mis en place de telle sorte que son axe 5B s'étende perpendiculairement à ce plan W.

Dans la mesure où le recours aux deux seuls points précités Pᵤₚ et P_{down} est insuffisant pour fournir toutes les caractéristiques spatiales du plan d'implantation W, l'ordinateur 2 peut à cette fin soit utiliser des renseignements directement fournis par le chirurgien, soit orienter dans l'espace le plan W passant par les points Pᵤₚ et P_{down} à l'aide de l'angle de Lévigne, en intégrant à l'ordinateur 2 une base de données relatives à la définition de cet angle, telles que fournies par la littérature orthopédique.

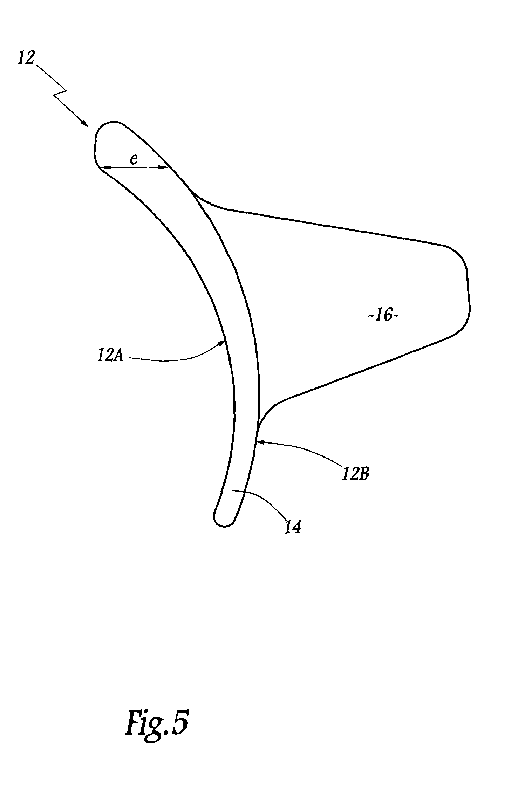

Sur la figure 5 est représenté un composant glénoïdien 12 d'une prothèse d'épaule, qui, à la différence du composant 5 de la figure 1, n'est pas une pièce pré-existante, notamment disponible dans une gamme d'implants homothétiques. Le composant 12 comporte un corps d'implant massif 14, notamment de nature métallique ou synthétique, qui présente une forme globale de cupule.

Sur son côté destiné à coopérer par articulation avec l'humérus d'un patient, portant notamment un composant prothétique huméral de la prothèse d'épaule, le corps 14 présente une face concave 12A dont l'essentiel, voire la totalité comme ici, définit une surface articulaire adaptée pour s'articuler contre une surface sensiblement complémentaire, non représentée sur les figures, délimitée soit par l'extrémité supérieure anatomique d'un humérus, soit par le composant huméral de la prothèse d'épaule.

Sur son côté opposé, le corps d'implant 14 présente une face 12B qui, lorsque le composant 12 est implanté, est appuyé directement ou indirectement contre l'extrémité latérale osseuse d'une omoplate, préalablement préparée à cette fin. De manière connue en soi, la face 12B est avantageusement munie de moyens d'ancrage osseux dans l'omoplate, par exemple sous la forme d'une quille 16 comme sur la figure 5.

Le composant glénoïdien 12 a été fabriqué « sur mesure » pour l'omoplate S de la figure 1. Ainsi, la face articulaire 12A ne correspond pas à une géométrie standard pré-existante, mais reproduit la surface glénoïdienne théorique G' fournie par l'ordinateur 2 lors du troisième temps opératoire évoqué plus haut. Comme cette surface glénoïdienne théorique G' est une estimation fiable de la surface glénoïdienne avant dégénérescence g, on comprend que la face articulaire 12A est très proche, d'un point de vue géométrique, de cette surface g, ce qui explique l'utilisation de l'expression précitée « sur mesure ». Le comportement articulaire du composant glénoïdien 12 au niveau de sa face 12A est ainsi quasi identique au comportement naturel de l'épaule du patient, avant la dégénérescence de son omoplate S.

Avantageusement, la face d'appui 12B ne correspond pas non plus à une géométrie standard pré-existante, mais tient compte de l'état de dégénérescence de la surface glénoïdienne G de l'omoplate S. En particulier, la partie d'extrémité supérieure de cette face 12B est conformée pour tenir compte de l'usure prononcée dans la partie haute de la surface glénoïdienne G. Pour ce faire, l'épaisseur du corps d'implant 14, c'est-à-dire sa dimension suivant une direction médio-latérale, est plus grande dans sa partie d'extrémité supérieure du corps que dans le reste du corps, la valeur maximale de cette épaisseur au niveau de la partie d'extrémité haute du corps 14 étant noté e sur la figure 5. La variation de l'épaisseur du corps d'implant 14 est déterminée, par exemple à l'aide de l'ordinateur 2, à partir des données de cartographie correspondant à la partie de surface la plus haute G₃. De cette façon, lorsque l'extrémité latérale de l'omoplate S est préparée en vue de l'implantation du composant 12, le chirurgien ne dégage, dans la région inférieure de la surface glénoïdienne G, ici au niveau des parties de surface G₁ et G₂, qu'une quantité de matière osseuse limitée, juste suffisante pour tenir compte de la faible épaisseur de la partie basse du corps d'implant 14, tandis que la partie haute de ce corps d'implant est dimensionnée en épaisseur pour s'ajuster sensiblement à la partie de surface G₃. Autrement dit, contrairement à ce qui est pratiqué couramment, le chirurgien ne cherche pas à « niveler » la surface glénoïdienne G, lors de sa préparation, sur sa région haute la plus usée, ce qui conduirait à entamer de manière excessive des zones osseuses saines de l'omoplate et à médialiser le composant glénoïdien, sauf à sur-épaissir le corps d'implant.

En pratique, la mise en forme de la matière constituant le composant glénoïdien 12 est réalisée par des techniques connues en soi. Dans le cas d'un composant glénoïdien métallique, ce dernier peut être coulé puis usiné. Si une matière plastique est utilisée, elle est généralement coulée puis, le cas échéant, rectifiée. Dans tous les cas, les faces 12A et, avantageusement, 12B sont travaillées de manière fine, pour les ajuster respectivement à la surface glénoïdienne théorique G' et à l'extrémité latérale préalablement préparée de l'omoplate S.

Divers aménagements et variantes au dispositif d'implantation 1, à la méthode d'implantation et à la méthode de fabrication du composant 12, décrits ci-dessus sont par ailleurs envisageables. A titre d'exemples :
- les moyens de repérage de l'os de l'omoplate S et/ou du palpeur 10 ne sont pas limités à des marqueurs réfléchissant l'infrarouge ; des marqueurs sensibles aux ultrasons ou à des champs électromagnétiques sont par exemple utilisables ;
- la nature géométrique des portions de sphères imaginaires G'₁, G'₂ et G'₃ peut être généralisée à celle de portions d'ellipsoïde, notamment afin de modéliser ces portions de surface au plus près des parties correspondantes G₁, G₂ et G₃ de la surface glénoïdienne G ; d'ailleurs, notamment dans ce cas, il est possible de ne prévoir la modélisation que d'une seule portion d'ellipsoïde, qui coïncide sensiblement avec une partie la plus basse de la surface glénoïdienne G et qui constituera alors, aux fins de la méthode, la surface glénoïdienne théorique G' ;
- le fait de déterminer le plan d'implantation W peut être rendu optionnel lorsque l'ordinateur 2 est à même de guider dans l'espace un outil d'implantation du composant glénoïdien 5 en vue que la surface articulaire 5A de ce composant soit positionnée en coïncidence avec la surface glénoïdienne théorique G', en tenant compte du point de référence bas P_{down} ;
- l'étendue verticale de l'unique partie de surface de glène la plus basse ou celle de chacune des différentes parties de surface de glène se succédant de bas en haut peut être pré-établie, choisie arbitrairement par le chirurgien en l'indiquant à l'ordinateur 2, ou bien calculée par l'ordinateur, notamment par une formule de rapport entre l'étendue de la partie considérée et l'étendue totale de la glène G ; et/ou
- davantage que trois portions de sphère, voire d'ellipsoïde, imaginaires peuvent être modélisées à partir d'autant de parties de surface successives, suivant la verticale, de la surface glénoïdienne dégénérative G, du moment que la surface glénoïdienne théorique sphérique G', permettant de déterminer la position dans l'espace du point de référence haut P_{up,} est calculée à partir d'uniquement le ou des groupes de données de cartographie correspondant respectivement à la partie de surface la plus basse ou à des parties de surfaces situées le plus bas.

## Revendications

1. Procédé de modélisation d'une surface glénoïdienne d'une omoplate, **caractérisé en ce qu'**il comporte des étapes successives consistant à :
- disposer de données de cartographie représentatives de points appartenant à la surface glénoïdienne (G) à modéliser,
- distinguer, parmi les données de cartographie, un premier groupe de données de cartographie correspondant à une première partie (G₁) seulement de la surface glénoïdienne (G), cette première partie de surface étant située le plus bas suivant la direction verticale associée à l'omoplate (S),
- déterminer par calcul, exclusivement à partir du premier groupe de données de cartographie, une première portion d'ellipsoïde imaginaire (G'₁) qui coïncide sensiblement avec la première partie de surface (G₁), et
- fournir une surface glénoïdienne théorique (G') à partir de la première portion d'ellipsoïde (G'₁).

2. Procédé suivant la revendication 1, **caractérisé en ce que** la surface glénoïdienne théorique (G') est constituée de la première portion d'ellipsoïde (G'₁).

3. Procédé suivant la revendication 1, **caractérisé en ce que**, parmi les données de cartographie, on distingue un ou plusieurs groupes de données de cartographie autre(s) que le premier groupe de données de cartographie, correspondant respectivement à autant de partie(s) de surface (G₂, G₃) de la surface glénoïdienne (G), distincte(s) de la première partie de surface (G₁) et disposée(s), suivant la direction verticale associée à l'omoplate, de manière successive à cette première partie de surface et, le cas échéant, les unes aux autres,
et **en ce qu'**on détermine par calcul, à partir du ou des autre(s) groupe(s) de données de cartographie, respectivement une ou plusieurs portion(s) d'ellipsoïde imaginaire (G'₂, G'₃) autre que la première portion d'ellipsoïde (G'₁), cette ou ces autres portion(s) d'ellipsoïde coïncidant sensiblement avec respectivement la ou les autres partie(s) de surface correspondante(s) (G₂, G₃),
et **en ce qu'**on fournit la surface glénoïdienne théorique (G') à partir de la première portion d'ellipsoïde (G'₁) et de l'autre ou d'au moins une (G'₂) des autres portion(s) d'ellipsoïde.

4. Procédé suivant l'une quelconque des revendications précédentes, **caractérisé en ce que**, dans le cas où la première portion d'ellipsoïde (G'₁) et, le cas échéant, la ou les autres portion(s) d'ellipsoïde (G'₂, G'₃) correspondent à des portions de sphère, on détermine la ou chaque portion de sphère en calculant la position de son centre (C'₁, C'₂, C'₃) et la valeur de son rayon (r'₁, r'₂, r'₃).

5. Procédé suivant les revendications 3 et 4 prises ensemble, **caractérisé en ce que** la surface glénoïdienne théorique (G') est sphérique, la position de son centre (C') et la valeur de son rayon (r') étant calculées respectivement comme le barycentre des centres (C'₁, C'₂) et la moyenne des rayons (r'₁, r'₂) de la première portion d'ellipsoïde (G'₁) et de l'autre ou d'au moins une (G'₂) des autres portion(s) d'ellipsoïde correspondant à la ou aux partie(s) de surface (G₂) située(s) le plus bas.

6. Procédé suivant l'une quelconque des revendications précédentes, **caractérisé en ce qu'**on détermine la première portion d'ellipsoïde (G'₁) et, le cas échéant, la ou les autres portion(s) d'ellipsoïde (G'₂, G'₃), en calculant les caractéristiques spatiales de la ou chaque portion d'ellipsoïde de telle sorte que cette portion d'ellipsoïde inclut, à un écart multidirectionnel préfixé près, le plus grand nombre de points de la surface glénoïdienne (G), qui sont représentés par les données de cartographie du groupe de données associé exclusivement à cette portion d'ellipsoïde.

7. Dispositif chirurgical (1) d'implantation d'un composant glénoïdien (5 ; 12) d'une prothèse d'épaule, comportant :
- des moyens de repérage (2, 3, 4, 6) pour repérer dans l'espace l'omoplate (S) d'un patient opéré,
- des moyens de cartographie (2, 10) pour cartographier la surface glénoïdienne (G) de cette omoplate (S),
- des moyens de modélisation (2) pour mettre en oeuvre le procédé de modélisation conforme à l'une quelconque des revendications 1 à 6 et obtenir ainsi une surface glénoïdienne théorique (G') à partir des données de cartographie de la surface glénoïdienne (G) fournies par les moyens de cartographie (2, 10),
- des premiers moyens de détermination (2) pour déterminer la position dans l'espace d'un point de référence bas (P_{down}) pour implanter le composant glénoïdien (5 ; 12), à partir des données de cartographie fournies exclusivement au niveau de l'extrémité inférieure de la surface glénoïdienne (G) par les moyens de cartographie (2, 10), et
- des moyens d'implantation (2) pour fournir une configuration spatiale d'implantation du composant glénoïdien (5 ; 12), au moins à partir de la surface glénoïdienne théorique (G') et du point de référence bas (P_{down}).

8. Dispositif suivant la revendication 7, **caractérisé en ce que** les premiers moyens de détermination (2) sont adaptés pour positionner le point de référence bas (P_{down}) à l'emplacement du point (P₁) de la surface glénoïdienne (G) cartographié par les moyens de cartographie (2, 10) et situé le plus bas.

9. Dispositif suivant l'une des revendications 7 ou 8, **caractérisé en ce qu'**il comporte en outre des deuxièmes moyens de détermination (2) pour déterminer la position dans l'espace d'un point de référence haut (Pₑₚ) pour implanter le composant glénoïdien (5 ; 12), à partir, d'une part, des données de cartographie obtenues exclusivement au niveau de l'extrémité supérieure de la surface glénoïdienne (G) par les moyens de cartographie (2, 10) et, d'autre part, de la surface glénoïdienne théorique (G'),
et **en ce que** les moyens d'implantation (2) sont adaptés pour fournir la position dans l'espace d'un plan (W) d'implantation du composant glénoïdien (5 ; 12), qui passe par les points de référence haut (Pₑₚ) et bas (P_{down}).

10. Dispositif suivant la revendication 9, **caractérisé en ce que** les deuxièmes moyens de détermination (2) sont adaptés pour positionner le point de référence haut (Pᵤₚ) à l'intersection entre la surface glénoïdienne théorique (G') et une droite radiale à cette surface, passant par le point (P₂) de la surface glénoïdienne (G), cartographié par les moyens de cartographie (2, 10) et situé le plus haut.

11. Dispositif suivant l'une quelconque des revendications 7 à 10, **caractérisé en ce qu'**il comporte en outre des moyens d'affichage (8) pour afficher, à la fois, la surface glénoïdienne (G) telle que cartographiée par les moyens de cartographie (2, 10), le point de référence bas (P_{down}) et au moins certaines caractéristiques géométriques (C') de la surface glénoïdienne théorique (G'), ainsi que, le cas échéant, le point de référence haut (Pᵤₚ) et le plan d'implantation (W).

12. Procédé de fabrication d'un composant glénoïdien (12) d'une prothèse d'épaule (10) pour une omoplate (S), dans lequel on modélise la surface glénoïdienne (G) de l'omoplate conformément au procédé de modélisation selon l'une quelconque des revendications 1 à 6, et
dans lequel on conforme la face articulaire (12A) d'un corps d'implant (14), destinée à s'articuler contre une surface humérale sensiblement complémentaire, de manière qu'au moins une partie de cette face articulaire reproduise la surface glénoïdienne théorique (G') fournie par le procédé de modélisation.

13. Procédé suivant la revendication 12, **caractérisé en ce que** qu'on détermine l'épaisseur (e), suivant une direction médio-latérale, de la partie d'extrémité haute du corps d'implant (14), à partir de la surface glénoïdienne théorique (G') et des données de cartographie correspondant à une partie seulement (G₃) de la surface glénoïdienne (G), cette partie de surface étant située le plus haut suivant la direction verticale associée à l'omoplate (S).

## Patentansprüche

1. Modellierungsverfahren einer Gelenkoberfläche eines Schulterblatts, **dadurch gekennzeichnet, dass** es die aufeinanderfolgenden Schritte aufweist, die darin bestehen:
- Zurverfügungstellen von Kartografiedaten, die für zu der zu modellierenden Gelenkoberfläche (G) gehörenden Punkte repräsentativ sind,
- Auswählen einer ersten Kartografiedatengruppe entsprechend nur einem ersten Teil (G₁) der Gelenkoberfläche (G) aus den Kartografiedaten, wobei dieser erste Teil der Oberfläche gemäß der dem Schulterblatt (S) zugeordneten vertikalen Richtung zuunterst angeordnet ist,
- Bestimmen eines ersten imaginären Ellipsoidbereichs (G'₁) durch Berechnung ausschließlich ausgehend von der ersten Kartografiedatengruppe, wobei der imaginäre Ellipsoidbereich im Wesentlichen mit dem ersten Oberflächenteil (G₁) übereinstimmt, und
- Liefern einer theoretischen Gelenkfläche (G') ausgehend von dem ersten Ellipsoidbereich (G'₁).

2. Verfahren nach Anspruch 1, **dadurch gekennzeichnet, dass** die theoretische Gelenkoberfläche (G') aus dem ersten Ellipsoidbereich (G'₁) gebildet ist.

3. Verfahren nach Anspruch 1, **dadurch gekennzeichnet, dass** aus den Kartografiedaten eine oder mehrere Kartografiedatengruppen, die nicht die erste Kartografiedatengruppe ist bzw. sind, ausgewählt werden, die jeweils so vielen Oberflächenteilen (G₂, G₃) der Gelenkoberfläche (G), unterschiedlich zu dem ersten Oberflächenteil (G₁), entsprechen und die gemäß der dem Schulterblatt zugeordnete Richtung in aufeinanderfolgender Weise von diesem ersten Oberflächenteil aus und gegebenenfalls der eine Oberflächenteil an den anderen angeordnet sind,
und dass aus der oder den anderen Kartografiedatengruppen jeweils ein oder mehrere imaginäre Ellipsoidbereiche (G'₂, G'₃), die nicht der erste Ellipsoidbereich (G'₁) ist bzw. sind, bestimmt werden, wobei dieser oder diese anderen Ellipsoidbereiche im Wesentlichen mit jeweils dem oder den anderen korrespondierenden Oberflächenteilen (G₂, G₃) übereinstimmen,
und dass die theoretische Gelenkoberfläche (G') ausgehend von dem ersten Ellipsoidbereich (G'₁) und dem anderen oder mindestens einem (G'₂) der anderen Ellipsoidbereiche geliefert wird.

4. Verfahren nach einem beliebigen der vorhergehenden Ansprüche, **dadurch gekennzeichnet, dass** im Fall, in dem der erste Ellipsoidbereich (G'₁) und gegebenenfalls der oder die anderen Ellipsoidbereiche (G'₂, G'₃) Kugelbereichen entsprechen, der oder jeder Kugelbereich durch Berechnen der Position seiner Mitte (C'₁, C'₂, C'₃) und des Wertes seines Radius (r'₁, r'₂, r'₃) bestimmt werden.

5. Verfahren nach den Ansprüchen 3 und 4 zusammengenommen, **dadurch gekennzeichnet, dass** die theoretische Gelenkoberfläche (G') kugelförmig ist, wobei die Position ihrer Mitte (C') und der Wert ihres Radius (r') jeweils berechnet werden als Schwerpunkt der Mitten (C'₁, C'₂) und als Mittelwert der Radien (r'₁, r'₂) des ersten Ellipsoidbereichs (G'₁) und des anderen oder mindestens eines (G'₂) der anderen Ellipsoidbereiche korrespondierend zu dem oder den Oberflächenteilen (G₂), die zuunterst liegen.

6. Verfahren nach einem beliebigen der vorhergehenden Ansprüche, **dadurch gekennzeichnet, dass** der erste Ellipsoidbereich (G'₁) und gegebenenfalls der oder die anderen Ellipsoidbereiche (G'₂, G'₃) bestimmt werden, indem die Raumkenngrößen des oder jedes Ellipsoidbereichs derart berechnet werden, dass dieser Ellipsoidbereich in einem vorbestimmten nahen Abstand in mehreren Richtungen die größte Anzahl an Punkten der Gelenkoberfläche (G) einschließt, die durch die Kartografiedaten der Datengruppe repräsentiert werden, die ausschließlich diesem Ellipsoidbereich zugeordnet ist.

7. Chirurgische Vorrichtung (1) zur Implantation einer Gelenkkomponente (5; 12) einer Schulterprothese, umfassend:
- Ortungsmittel (2, 3, 4, 6) zum Orten des Schulterblatts (S) eines operierten Patienten im Raum,
- Kartografiemittel (2, 10), um die Gelenkoberfläche (G) dieses Schulterblattes (S) zu kartografieren,
- Modellierungsmittel (2), um das Modellierverfahren entsprechend einem beliebigen der Ansprüche 1 bis 6 umzusetzen und so eine theoretische Gelenkoberfläche (G') aus den Kartografiedaten der Gelenkoberfläche (G) zu erhalten, die von den Kartografiemitteln (2, 10) geliefert werden,
- erste Bestimmungsmittel (2), um die Position eines unteren Referenzpunktes (P_{down}) im Raum zum Implantieren der Gelenkkomponenten (5; 12) zu bestimmen, aus den Kartografiedaten, die ausschließlich am unteren Ende der Gelenkoberfläche (G) von den Kartografiemittel (2, 10) geliefert wurden, und
- Implantationsmittel (2) zum Liefern einer Implantationsraumkonfiguration der Gelenkkomponente (5; 12) mindestens aus der theoretischen Gelenkoberfläche (G') und dem unteren Referenzpunkt (P_{down}).

8. Vorrichtung nach Anspruch 7, **dadurch gekennzeichnet, dass** die ersten Bestimmungsmittel (2) angepasst sind, den unteren Referenzpunkt (P_{down}) an die Stelle des Punktes (P₁) der Gelenkoberfläche (G) zu positionieren, der durch die Kartografiemittel (2, 10) kartografiert wurde und zuunterst liegt.

9. Vorrichtung nach einem der Ansprüche 7 oder 8, **dadurch gekennzeichnet, dass** sie außerdem zweite Bestimmungsmittel (2) aufweist, um die Position eines oberen Referenzpunkts (Pₑₚ) im Raum zum Implantieren der Gelenkkomponenten (5; 12) zu bestimmen, einerseits aus den Kartografiedaten, die ausschließlich an dem oberen Ende der Gelenkoberfläche (G) von den Kartografiemitteln (2, 10) und andererseits von der theoretischen Gelenkoberfläche (G') erhalten wurden, und dass die Implantationsmittel (2) angepasst sind, die Position einer Implantationsebene (W) der Gelenkoberfläche (5; 12) im Raum zu liefern, die durch den oberen (Pₑₚ) und den unteren (P_{down}) Referenzpunkt hindurchgeht.

10. Vorrichtung nach Anspruch 9, **dadurch gekennzeichnet, dass** die zweiten Bestimmungsmittel (2) angepasst sind, den oberen Referenzpunkt (Pᵤₚ) am Schnittpunkt zwischen der theoretischen Gelenkoberfläche (G') und einer radialen Geraden zu dieser Oberfläche zu positionieren, die durch den Punkt (P₂) der Gelenkoberfläche (G) hindurchgeht, der von den Kartografiemitteln (2, 10) kartografiert wurde und zuoberst liegt.

11. Vorrichtung nach einem beliebigen der Ansprüche 7 bis 10, **dadurch gekennzeichnet, dass** sie außerdem Anzeigemittel (8) zum gleichzeitigen Anzeigen der Gelenkoberfläche (G), wie von den Kartografiemitteln (2, 10) kartografiert, des unteren Referenzpunkts (P_{down}) und mindestens bestimmter geometrischer Kenngrößen (C') der theoretischen Gelenkoberfläche (G') sowie gegebenenfalls des oberen Referenzpunkts (Pᵤₚ) und der Implantationsebene (W) umfasst.

12. Verfahren zur Herstellung einer Gelenkkomponente (12) einer Schulterprothese (10) für ein Schulterblatt (S), bei dem die Gelenkoberfläche (G) des Schulterblatts gemäß dem Verfahren zum Modellieren nach einem beliebigen der Ansprüche 1 bis 6 modelliert wird, und bei dem die Gelenkstirnfläche (12A) eines Implantatkörpers (14), die vorgesehen ist, gelenkig gegen eine im Wesentlichen komplementäre Humeralfläche angeordnet zu werden, angepasst wird, derart, dass mindestens ein Teil dieser Gelenkstirnfläche die theoretische Gelenkfläche (G') reproduziert, die durch das Modellierungsverfahren geliefert wird.

13. Verfahren nach Anspruch 12, **dadurch gekennzeichnet, dass** die Dicke (e) des oberen Endes des Implantatkörpers (14) gemäß einer mediolateralen Richtung aus der theoretischen Gelenkfläche (G') und den Kartografiedaten entsprechend nur einem Teil (G₃) der Gelenkfläche (G) bestimmt wird, wobei dieser Oberflächenteil gemäß der dem Schulterblatt (S) zugeordneten vertikalen Richtung am höchsten liegt.

## Claims

1. Method for modelling a glenoid surface of a scapula, **characterised in that** it comprises successive steps consisting of:
• having available mapping data representing points belonging to the glenoid surface (G) to be modelled,
• selecting from the mapping data a first group of mapping data corresponding to a first part (G₁) only of the glenoid surface (G), wherein this first surface part is located at the lowest point in the vertical direction in relation to the scapula (S),
• determining by calculation exclusively from the first group of mapping data a first imaginary ellipsoid portion (G'₁) that coincides substantially with the first surface part (G₁), and
• obtaining a theoretical glenoid surface (G') from the first ellipsoid portion (G'₁).

2. Method according to claim 1, **characterised in that** the theoretical glenoid surface (G') is formed from the first ellipsoid portion (G'₁).

3. Method according to claim 1, **characterised in that** one or more groups of mapping data are distinguished from the mapping data, other than the first group of mapping data respectively corresponding to as many surface parts (G₂, G₃) of the glenoid surface (G), which is/are distinct from the first surface part (G₁) and is/are arranged in succession in the vertical direction in relation to the scapula on this first surface part and, where applicable, one on top of the other,
and **in that** one or more imaginary ellipsoid portions (G'₂, G'₃) other than the first ellipsoid portion (G'₁) are respectively determined by calculation from the or other groups of mapping data, wherein this or these other ellipsoid portions coincide substantially with the or the other corresponding surface parts (G₂, G₃),
and **in that** the theoretical glenoid surface (G') is obtained from the first ellipsoid portion (G'₁) and the other or at least one (G'₂) of the other ellipsoid portions.

4. Method according to any one of the preceding claims, **characterised in that** in the case where the first ellipsoid portion (G'₁) and, where applicable, the or the other ellipsoid portion or portions (G'₂, G'₃) correspond to sphere portions, the or each sphere portion is determined by calculating the position of its centre (C'₁, C'₂, C'₃) and the value of its radius (r'₁, r'₂, r'₃).

5. Method according to claims 3 and 4 taken in combination, **characterised in that** the theoretical glenoid surface (G') is spherical, wherein the position of its centre (C') and the value of its radius (r') are respectively calculated as the centre of mass of the centres (C'₁, C'₂) and the mean of the radii (r'₁, r'₂) of the first ellipsoid portion (G'₁) and the other or at least one (G'₂) of the other ellipsoid portion or portions corresponding to the surface part or parts (G₂) located at the lowest point.

6. Method according to any one of the preceding claims, **characterised in that** the first ellipsoid portion (G'₁) and, where applicable, the or the other ellipsoid portions (G'₂, G'₃) is/are determined by calculating the spatial characteristics of the or each ellipsoid portion in such a way that, with a preset multidirectional deviation, this ellipsoid portion represents the largest number of points of the glenoid surface (G) that are represented by the mapping data of the group of data associated exclusively with this ellipsoid portion.

7. Surgical device (1) for implanting a glenoid component (5; 12) of a shoulder prosthesis comprising:
• locating means (2, 3, 4, 6) for spatially locating the scapula (S) of a patient undergoing an operation,
• mapping means (2, 10) for mapping the glenoid surface (G) of this scapula (S),
• modelling means (2) for implementing the modelling method according to any one of claims 1 to 6 and thus obtaining a theoretical glenoid surface (G') from the mapping data of the glenoid surface (G) provided by the mapping means (2, 10),
• first determination means (2) for determining the spatial position of a low reference point (P_{down}) for implanting the glenoid component (5; 12) from the mapping data provided exclusively at the level of the lower end of the glenoid surface (G) by the mapping means (2, 10), and
• implantation means (2) for obtaining a spatial implantation configuration of the glenoid component (5; 12) at least from the theoretical glenoid surface (G') and the low reference point (P_{down}).

8. Device according to claim 7, **characterised in that** the first determination means (2) are adapted to position the low reference point (P_{down}) at the placement of the point (P₁) of the glenoid surface (G) mapped by the mapping means (2, 10) and located at the lowest point.

9. Device according to one of claims 7 or 8, **characterised in that** it additionally comprises second determination means (2) for determining the spatial position of a high reference point (Pᵤₚ) for implanting the glenoid component (5; 12) from the mapping data obtained exclusively at the level of the upper end of the glenoid surface (G) by the mapping means (2, 10), on the one hand, and from the theoretical glenoid surface (G'), on the other hand,
and **in that** the implantation means (2) are adapted to provide the spatial position of an implantation plane (W) of the glenoid component (5; 12), which passes through the high (Pᵤₚ) and low (P_{down}) reference points.

10. Device according to claim 9, **characterised in that** the second determination means (2) are adapted to position the high reference point (Pᵤₚ) at the intersection between the theoretical glenoid surface (G') and a line radial to this surface passing through the point (P₂) of the glenoid surface (G) mapped by the mapping means (2, 10) and located at the highest point.

11. Device according to any one of claims 7 to 10, **characterised in that** it additionally comprises display means (8) for simultaneously displaying the glenoid surface (G) as mapped by the mapping means (2, 10), the low reference point (P_{down}) and at least certain geometric characteristics (C') of the theoretical glenoid surface (G') and also, where applicable, the high reference point (Pᵤₚ) and the implantation plane (W).

12. Method for fabricating a glenoid component (12) of a shoulder prosthesis (10) for a scapula (S), in which the glenoid surface (G) of the scapula is modelled in accordance with the modelling method according to any one of claims 1 to 6, and
in which the articular face (12A) of an implant body (14) intended to articulate against a substantially complementary humeral surface is shaped in such a way that at least one part of this articular face reproduces the theoretical glenoid surface (G') obtained by the modelling method.

13. Method according to claim 12, **characterised in that** the thickness (e) in a mediolateral direction of the high end part of the implant body (14) is determined from the theoretical glenoid surface (G') and the mapping data corresponding to one part only (G₃) of the glenoid surface (G), wherein this surface part is located at the highest point in the vertical direction in relation to the scapula (S).
